# EUROPEAN PATENT SPECIFICATION

(11) **EP 3 352 570 B1**
(45) Date of publication and mention of the grant of the patent: **08.04.2020**
(21) Application number: 16794730.8
(22) Date of filing: 23.09.2016
(51) Int. Cl.: A01N 43/66, A01N 43/72, A01N 43/74, A01N 43/86, C07D 417/04, C07D 285/125

(54) **HETEROCYCLIC TRIFLUOROALKENYL COMPOUNDS HAVING A NEMATOCIDAL ACTIVITY, THEIR AGRONOMIC COMPOSITIONS AND USE THEREOF**
HETEROCYCLISCHE TRIFLUORALKENYLVERBINDUNGEN MIT NEMATIZIDER AKTIVITÄT, DEREN AGRONOMISCHE ZUSAMMENSETZUNGEN UND VERWENDUNG DAVON
COMPOSÉS DE TRIFLUOROALCÉNYLE HÉTÉROCYCLIQUES PRÉSENTANT UNE ACTIVITÉ NÉMATOCIDE, LEURS COMPOSITIONS AGRONOMIQUES ET UTILISATION ASSOCIÉE

(30) Priority: 23.09.2015 IT UB20153829
(43) Date of publication of application: 01.08.2018
(73) Proprietor: ISAGRO S.p.A., 20153 Milano (IT)
(72) Inventor: BELLANDI, Paolo, 17043 Carcare (SV) (IT); GUSMEROLI, Marilena, 20900 Monza (MB) (IT); SARGIOTTO, Chiara, 28066 Galliate (NO) (IT); BIANCHI, Daniele, 28043 Bellinzago Novarese (NO) (IT)
(74) Representative: De Gregori, Antonella
(86) International application number: PCT/IB2016/055685
(87) International publication number: WO 2017/002100

(56) References cited:
- EP-A1- 0 410 551
- WO-A1-01/02378
- WO-A1-02/06256
- WO-A1-95/24403
- WO-A1-99/52882
- JP-A- H11 140 063

## Description

The present invention relates to new heterocyclic trifluoroalkenyl compounds having formula (I):

Het-S(O)ₙ-(CH₂)ₘ-CF=CF₂ (I)

The present invention also relates to agronomic compositions containing said compounds having formula (I) and their use for the control of nematodes in agricultural crops.

### STATE OF THE ART

Heterocyclic compounds carrying a fluoroalkenyl chain have been described in literature for use as pesticides and, in particular, as nematocides.

These compounds are composed of three characteristic residues: an aromatic or non-aromatic heterocycle, a S(O)ₙ residue wherein n is an integer ranging from 0 to 2 and a fluoroalkenyl chain having general formula -(CH₂)ₘ-CX=CF₂ wherein m can range from 1 to 10 and X is a hydrogen or fluorine atom.

Patent application EP410551 describes thioethers having general formula (A) bearing a thiadiazole residue and a trifluorobutenyl chain.

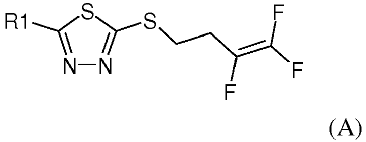

Patent application WO95/24403 describes heterocyclic difluorobutenyl compounds having general formula (B):

Het-S(O)ₙ-(CH₂)₂-CH=CF₂ (B)

Patent application WO01/02378 discloses thiazole derivatives having general formula (C) bearing a trifluorobutenyl chain:

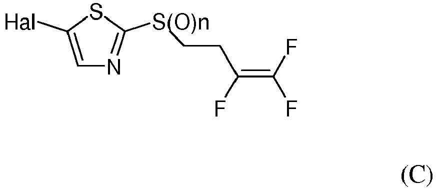

Patent application WO02/06256 describes heterocyclic compounds having general formula (D) bearing a fluoroalkenyl chain wherein m is an integer ranging from 3 to 10 and X represents a hydrogen atom, a halogen or an alkyl group:

Het-S(O)ₙ-(CH₂)ₘ-CX=CF₂ (D)

All of these compounds, however, are unsatisfactory in terms of nematocidal activity, as they are not able to effectively limit the attack of the parasite and reduce the formation of galls on the root system of the plant.

Furthermore, in various cases, these products have proved to be phytotoxic with respect to important agricultural crops, showing a significant necrosis of the leaves and stem, at the dosages that allow a good nematocidal activity to be obtained.

### DESCRIPTION

The Applicant has now surprisingly found new heterocyclic trifluoroalkenyl compounds having a high nematocidal activity also at low dosages, which at the same time are well tolerated by agricultural crops.

A first object of the present invention therefore relates to new heterocyclic trifluoroalkenyl compounds having Formula (I):

Het-S(O)ₙ-(CH₂)ₘ-CF=CF₂ (I)

wherein:
- Het represents an aromatic heterocyclic group, substituted, selected from: 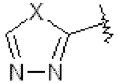
- X represents a sulfur atom;
- n represents 1 or 2;
- m represents 1 or 2.

Said heterocyclic group being substituted by groups selected from halogen, C₁-C₆ alkyl, C₁-C₆ haloalkyl, C₃-C₆ cycloalkyl, C₄-C₇ cycloalkylalkyl, C₁-C₆ alkoxyl, C₁-C₆ haloalkoxyl, C₁-C₆ alkylthio, C₁-C₆ haloalkylthio, C₁-C₆ alkylsulfinyl, C₁-C₆ alkylsulfonyl, C₁-C₆ alkoxycarbonyl, C₃-C₆ cycloalkoxycarbonyl, amino, N-C₁-C₆ alkylamino, N,N-C₂-C₁₂ dialkylamino, N-C₁-C₆ alkoxycarbonyl-amino, N-C₃-C₆ cycloalkylamino, N,N-C₆-C₁₂ dicycloalkylamino, N-C₃-C₆ cycloalkoxycarbonyl-amino, C₁-C₆ alkylaminocarbonyl, C₃-C₆ cycloalkylaminocarbonyl, a R₁R₂NCONR₃- group, formyl, carboxyl, cyano, an aryl optionally substituted, a benzyl, a heterocyclic aromatic penta- or hexa-atomic group, optionally benzocondensed or heterobicyclic, containing at least one heteroatom selected from oxygen, sulfur, nitrogen, possibly oxidized to N-oxide optionally substituted;
- R₁, R₂, R₃, the same or different, represent a hydrogen atom, a C₁-C₄ alkyl group or a C₃-C₆ cycloalkyl group.

Examples of halogen are: fluorine, chlorine, bromine, iodine.

Examples of linear or branched C₁-C₆ alkyls are methyl, ethyl, n-propyl, isopropyl, n-butyl, isobutyl, sec-butyl, tert-butyl, n-pentyl 3-methylbutyl, n-hexyl, 3,3-dimethylbutyl.

Examples of C₁-C₆ haloalkyls are: fluoromethyl, difluoromethyl, trifluoromethyl, chloromethyl, dichloromethyl, 2,2,2-trifluoroethyl, 1,1,2,2-tetrafluoroethyl, pentafluoroethyl, heptafluoropro-pyl, 4,4,4-trichlorobutyl, 4,4-difluoropentyl, 5,5-difluorohexyl.

Examples of linear or branched C₁-C₆ alkoxyls are methoxy, ethoxy, n-propoxy, isopropoxy, n-butoxy, isobutoxy, sec-butoxy, tert-butoxy, n-pentoxy, 3-methylbutoxy, hexyloxy, 3,3-dimethylbutoxy.

Examples of C₁-C₆ haloalkoxyls are fluoromethoxy, difluoromethoxy, trifluoromethoxy, chloro-methoxy, dichloromethoxy, 2,2,2-trifluoroethoxy, 1,1,2,2-tetra-fluoroethoxy, 1,1,2,3,3,3-hexafluoropropoxy, 4,4,4-trichlorobutoxy, 4,4-difluoropentoxy, 5,5-difluoro-hexyloxy.

Examples of C₁-C₆ alkylthios are methylthio, ethylthio, propylthio, isopropylthio, n-butylthio, isobutylthio, sec-butylthio, tert-butylthio, n-pentylthio, 3-methylbutylthio, n-hexylthio, 3,3-dimethylbutylthio.

Examples of C₁-C₆ haloalkylthios are difluoromethylthio, trifluoromethylthio, 1,1,2,2-tetrafluoro-ethylthio.

Examples of C₃-C₆ cycloalkyls are cyclopropyl, cyclobutyl, cyclopentyl, cyclohexyl.

Examples of C₃-C₈ halocycloalkyls are 2,2-dichloro-cyclopropyl, 2,2-difluorocyclopropyl, 2,2,3,3-tetrafluorocyclobutyl, 3,3-difluorocyclopentyl, 2-fluorocyclohexyl.

Examples of C₄-C₇ cycloalkylalkyls are methylcyclopropyl, methylcyclopentyl, ethylcyclopro-pyl, methylcyclohexyl.

Optionally substituted aryl refers to an aryl group that can have one or more substituents, the same or different, preferably selected from the following groups: halogen atoms, C₁-C₆ alkyl, C₁-C₆ haloalkyl, C₁-C₆ alkoxyl, C₁-C₆ haloalkoxyl, C₃-C₆ cycloalkoxyl, C₄-C₇ cycloalkylalkoxyl, phenoxyl, C₁-C₆ alkylthio, C₁-C₆ haloalkylthio, C₁-C₆ alkylsulfinyl, C₁-C₆ alkylsulfonyl, C₁-C₆ alkylamino, C₂-C₁₂ dialkylamino, cyano, C₂-C₇ alkoxycarbonyl, benzyloxycarbonyl, phenoxycarbonyl.

Aryl group refers to a phenyl or naphthyl group.

Aromatic heterocyclic group optionally substituted refers to a heterocyclic penta- or hexa-atomic ring, optionally benzocondensed or heterobicyclic, containing at least one heteroatom selected from nitrogen, oxygen and sulfur that can have one or more substituents, the same or different, preferably selected from the following groups: halogen atoms, C₁-C₆ alkyls, C₁-C₆ haloalkyls, C₁-C₆ alkoxyls, C₁-C₆ haloalkoxyls, C₃-C₆ cycloalkoxyls, C₄-C₇ cycloalkylalkoxyls, phenoxyls, C₁-C₆ thioalkoxyls, C₁-C₆ thiohaloalkoxyls, C₁-C₆ alkylsulfinyls, C₁-C₆ alkylsulfonyls, amino possibly substituted with one or two C₁-C₆ alkyl groups, cyano, nitro, carboxyl, C₂-C₅ alkoxycarbonyls, benzyloxycarbonyls, phenoxycarbonyls.

Examples of heterocyclic aromatic groups are: pyridyl, pyridyl N-oxide, pyrimidyl, pyridazyl, pyrazyl, furanyl, thiophenyl, pyrrolyl, oxazolyl, thiazolyl, isoxazolyl, isothiazolyl, oxadiazolyl, thiadiazolyl, pyrazolyl, imidazolyl, triazolyl, indolyl, benzofuranyl, benzothiophenyl, benzoxazolyl, benzothiazolyl, benzoxadiazolyl, benzothiadiazolyl, benzopyrazolyl, benzimidazolyl, benzotriazolyl, triazolopyridinyl, triazolopyrimidinyl, thiazolotriazolyl.

Specific examples of compounds having general formula (I) which are interesting for their nematocidal activity, are compounds wherein Het, n and m have the meanings indicated in Table 1:

Het-S(O)ₙ-(CH₂)ₘ-CF=CF₂ (I)

**Table 1**

| Nr | Het | n | m |
|---|---|---|---|
| 1 | 5-methyl-1,3,4-thiadiazole | 1 | 2 |
| 2 | 5-methyl-1,3,4-thiadiazole | 2 | 2 |
| 3 | 5-ethyl-1,3,4-thiadiazole | 1 | 2 |
| 4 | 5-ethyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 7 | 5-propyl-1,3,4-thiadiazole | 1 | 2 |
| 8 | 5-propyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |
| 11 | 5-cyclopropyl-1,3,4-thiadiazole | 1 | 2 |
| 12 | 5-cyclopropyl-1,3,4-thiadiazole | 2 | 2 |
| 13 | 5-cyclohexyl-1,3,4-thiadiazole | 1 | 2 |
| 14 | 5-cyclohexyl-1,3,4-thiadiazole | 2 | 2 |
| 15 | 5-trifluoromethyl-1,3,4-thiadiazole | 1 | 2 |
| 16 | 5-trifluoromethyl-1,3,4-thiadiazole | 2 | 2 |
| 17 | 5-difluoromethyl-1,3,4-thiadiazole | 1 | 2 |
| 18 | 5-difluoromethyl-1,3,4-thiadiazole | 2 | 2 |
| 19 | 5-phenyl-1,3,4-thiadiazole | 1 | 2 |
| 20 | 5-phenyl-1,3,4-thiadiazole | 2 | 2 |
| 21 | 5-chloro-1,3,4-thiadiazole | 1 | 2 |
| 22 | 5-chloro-1,3,4-thiadiazole | 2 | 2 |
| 23 | 5-bromo-1,3,4-thiadiazole | 1 | 2 |
| 24 | 5-bromo-1,3,4-thiadiazole | 2 | 2 |
| 25 | 5-thienyl-1,3,4-thiadiazole | 1 | 2 |
| 26 | 5-thienyl-1,3,4-thiadiazole | 2 | 2 |
| 27 | 5-isopentyl-1,3,4-thiadiazole | 1 | 2 |
| 28 | 5-isopentyl-1,3,4-thiadiazole | 2 | 2 |
| 29 | 5-(2,2-dimethylpropyl)-1,3,4-thiadiazole | 1 | 2 |
| 30 | 5-(2,2-dimethylpropyl)-1,3,4-thiadiazole | 2 | 2 |
| 31 | 5-(2-methylbutyl)-1,3,4-thiadiazole | 1 | 2 |
| 32 | 5-(2-methylbutyl)-1,3,4-thiadiazole | 2 | 2 |
| 33 | 5-methylcyclohexyl-1,3,4-thiadiazole | 1 | 2 |
| 34 | 5-methylcyclohexyl-1,3,4-thiadiazole | 2 | 2 |
| 35 | 5-methylcyclopropyl-1,3,4-thiadiazole | 1 | 2 |
| 36 | 5-methylcyclopropyl-1,3,4-thiadiazole | 2 | 2 |

Compounds having general formula (I) are preferred:

Het-S(O)ₙ-(CH₂)ₘ-CF=CF₂ (I)

wherein:
- Het, substituted, represents one of the following groups: 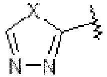
- substituents of the heterocycle are selected from halogen; C₁-C₆ alkyl; C₁-C₆ haloalkyl; C₃-C₆ cycloalkyl; formyl; optionally substituted aryl; benzyl; aromatic penta- or hexa-atomic heterocyclic group, optionally benzocondensed or heterobicyclic, containing at least one heteroatom selected from oxygen, sulfur, nitrogen or an optionally substituted N-oxide, preferably selected from oxygen, sulfur or nitrogen;
- X represents a sulfur atom;
- n represents 1 or 2.

Preferred compounds having general formula (I) are those wherein Het, n, and m have the following meanings:

| Nr | Het | n | m |
|---|---|---|---|
| 1 | 5-methyl-1,3,4-thiadiazole | 1 | 2 |
| 2 | 5-methyl-1,3,4-thiadiazole | 2 | 2 |
| 3 | 5-ethyl-1,3,4-thiadiazole | 1 | 2 |
| 4 | 5-ethyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |

Particularly preferred are compounds having general formula (I):

Het-S(O)ₙ-(CH₂)ₘ-CF=CF₂ (I)

wherein:
- Het, substituted, represents one of the following groups: 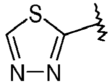
- the substituent of the heterocycle represents a C₁-C₆ alkyl group;
- m is equal to 2.

As is evident to skilled persons in the field, the compounds having general formula (I), when n has the value of 1, can be obtained in the form of two optical isomers.

An object of the present invention therefore relates to compounds having general formula (I) in racemic form, isomerically pure or mixtures thereof, possibly obtained during the preparation of compounds having general formula (I) or deriving from an incomplete separation of the same isomer, in any proportion.

The compounds having general formula (I) can be prepared starting from the corresponding thioether having formula (II) by oxidation reaction, as indicated in scheme 1.

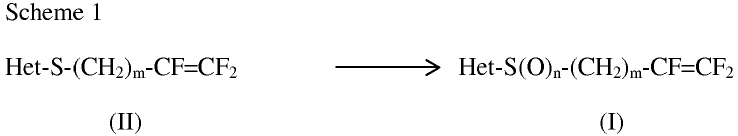

The reaction conditions, as described in WO 02/062770, envisage the use of an oxidizing agent in an appropriate solvent; oxidizing agents that can be used are organic peroxides, such as 4-chloro-perbenzoic acid, peracetic acid or inorganic peroxides such as, for example, hydrogen peroxide, potassium permanganate, sodium periodate.

The solvents used are preferably halogenated hydrocarbons such as dichloromethane or dichloroethane or chloroform, ethers such as dioxane or tetrahydrofuran, amides such as N,N-dimethylformamide or N-methyl-pyrrolidone, alcohols such as methanol, ethanol, propanol, isopropanol or ketones such as acetone or 2-butanone, acetic acid, water or mixtures thereof.

The reaction is carried out at a temperature ranging from 0 to 60°C, for a time ranging from 1 to 72 hours.

The compound having formula (II), when m has the values of 1 and 2, can be prepared as indicated in scheme 2, according to the method described in WO 03/037878.

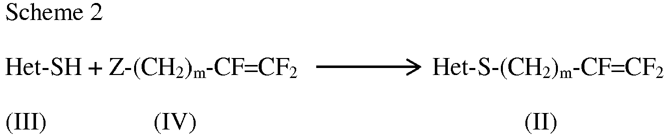

The reaction envisages treatment of the compound having formula (III) with a compound having Formula (IV), wherein Z represents an outgoing group, such as, for example, a halogen selected from Cl, Br, I, or a *p*-toluenesulfonate or trifluoromethanesulfonate group, in the presence of an organic or inorganic base such as, for example, triethylamine, pyridine, sodium acetate, potassium or calcium bicarbonate, sodium or potassium hydroxide, in a suitable solvent such as dichloroethane, chloroform or methylene chloride, at a temperature ranging from room temperature to the reflux temperature of the solvent selected.

When the compounds having Formula (III) are not commercial, they can be easily obtained according to methods known in organic chemistry, as described in Chemistry of Heterocyclic Compounds.

The compounds having Formula (IV), when Z represents a halogen atom, are commercial products. Alternatively, the compounds having Formula (IV), when Z represents a *p*-toluenesulfonate or trifluoromethanesulfonate group, can be obtained from the corresponding alcohols (V) according to what is described in Theodora W: Greene "Protective Groups in Organic Synthesis" Third Edition pages 198-199, as indicated hereunder in reaction scheme 3.

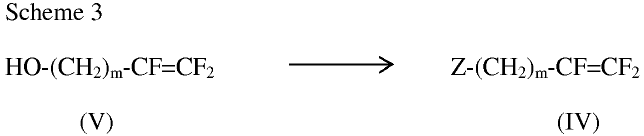

Alternatively, the compound having Formula (II) can be prepared by treatment of a compound having Formula (IV), with Z representing a halogen atom, with the salt having Formula (VII) suitably hydrolyzed to thiol, as indicated in reaction scheme 4.

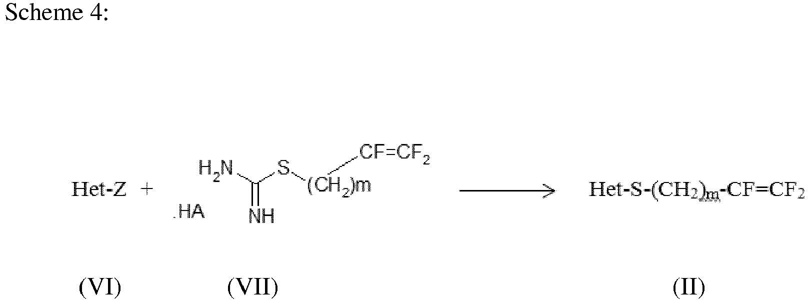

The reaction envisages a hydrolysis in situ of a compound having Formula (VII) in a solvent such as, for example, dioxane, tetrahydrofuran, dichloromethane, chloroform, toluene or in a mixture thereof with water in the presence or absence of an inorganic base such as sodium or potassium carbonate, sodium or potassium bicarbonate, sodium or potassium hydroxide, at room temperature, to give the corresponding thiol and subsequent treatment with a compound having Formula (VI), according to what is described in US 2005/215797. The thiol, obtained by the hydrolysis of (VII), can be possibly isolated and treated with (VI) in a subsequent step.

When the compounds having Formula (VI) are not commercial products, they can be easily obtained according to methods known in organic chemistry, as described in Chemistry of Heterocyclic Compounds.

As already indicated, the compounds having general formula (I) have a high nematocidal activity and do not show any phytotoxicity with respect to the crops of application. These features make them suitable for use in the agrarian field in defence against nematodes.

A further object of the present invention therefore relates to the use of compounds having Formula (I) for the control of nematodes.

Examples of nematodes that can be effectively limited with the compounds having Formula (I) are Pratylenchus spp, Globodera spp, Heterodera spp, Meloidogyne spp, Aphelenchoides spp, Radopholus Similis, Ditylenchus Dipsaci, Tylenchulus Semipenetrans, Longidorus spp, Xiphinema spp, Trichodorus spp, Bursaphelenchus spp, etc.

The compounds having Formula (I) are capable of exerting a nematocidal action of both a curative and preventive nature and have an extremely low or zero phytotoxicity with respect to the crops treated.

For practical uses in agriculture, it is often preferable to use the compounds of the present invention suitably formulated in agronomic compositions comprising one or more compounds having Formula (I) and agronomically acceptable co-formulants.

A further object of the present invention therefore relates to nematocidal agronomic compositions comprising one or more compounds having Formula (I), a solvent and/or solid, liquid or liquefied diluent, possibly one or more surfactants and other agronomically acceptable co-formulants.

Compositions can be used in the form of dry powders, wettable powders, emulsifiable concentrates, micro-emulsions, pastes, granulates, solutions, suspensions, fumigants, etc.: the selection of the type of composition depends on the specific use.

The compositions are prepared according to known methods, for example by diluting or dissolving the active substance with a solvent medium and/or solid diluent, possibly in the presence of surfactants.

Kaolin, alumina, silica, talc, bentonite, gypsum, quartz, dolomite, attapulgite, montmorillonite, diatomaceous earth, cellulose, starch, etc.., can be used as inert solid diluents, or carriers.

Inert liquid diluents that can be used are water, or organic solvents such as aromatic hydrocarbons (xylols, blends of alkyl benzenes, etc.), aliphatic hydrocarbons (hexane, cyclohexane, etc.) halogenated aromatic hydrocarbons (chlorobenzene, etc.), alcohols (methanol, propanol, butanol, octanol, etc.), esters (isobutyl acetate, etc.), ketones (acetone, cyclohexanone, acetophenone, isophorone, ethylamylketone etc.), or vegetable or mineral oils or mixtures thereof, etc.

Propellant gases such as butane, propane, halogenated hydrocarbons, nitrogen or carbon dioxide can be used as liquefied diluents or liquefied substances that gasify at room temperature and atmospheric pressure.

Surfactants that can be used are wetting and emulsifying agents of the non-ionic type (polyethoxylated alkyl phenols, polyethoxylated fatty alcohols, etc.), of the anionic type (alkylbenzenesulfonates, alkylsulfonates, etc.), of the cationic type (alkyl ammonium quaternary salts, etc.). Dispersing agents can also be added (for example lignin and its salts, cellulose derivatives, alginates, etc..), stabilizers (for example antioxidants, UV absorbers, etc.).

The concentration of active compound having Formula (I) in the above compositions can vary within a wide range and depends on various factors. It varies in relation to the active compound having Formula (I), the applications for which said compositions are destined, the environmental conditions and the type of formulation adopted. The concentration of active compound having Formula (I) generally ranges from 0.1 to 90% by weight with respect to the total weight of the composition, preferably from 0.5 to 90% by weight.

The compounds of the present invention as such or formulated can be used in a mixture with other active ingredients such as, for example, insecticides, acaricides, nematocides other than those having Formula (I), herbicides, fungicides, bactericides, fertilizers and biostimulants, etc. for broadening their spectrum or preventing resistance.

In some cases, the mixtures thus obtained have a synergic effect between the components, which causes the mixture, for example, to exert a higher activity with respect to that of the single elements of which it is composed.

Examples of insecticides, acaricides, nematocides that can be added to the compositions containing one or more compounds having general formula (I) are the following: abamectin, acetamiprid, acrinathrin, alphacypermethrin, alphamethrin, azadirachtin, Bacillus subtilis, Bacillus thuringiensis, Beauveria bassiana, betacyfluthrin, bifenazate, bifenthrin, buprofezin, chlorpyrifos, chlorpyrifos M, clofentezine, cyhalothrin, cyhexatin, cypermethrin, cyromazine, chloropicrin,clorantranilipide, clotianidin, deltamethrin, diflubenzuron, dimethoat, dazonet, sulfuryl difluoride, dimethyldisulfide, emamectin, esfenvalerate, ethoprophos, etofenprox, etoxazole, fenamiphos, fenazaquin, fenoxycarb, fenpyroximate, fipronil, fluazinam, flufenoxuron, fluvalinate, fosthiazate, formentanate, flonicamid, formet, viruses, hexythiazox, imidaclopridi, indoxacarb, lambda-cyhalothrin, lufenuron malathion, metaldehyde, methamidophos, Metharhizium spp, methiocarb, methomyl, methoxyfenozide, milbemectin, metaflumizone, metam sodium, metam potassium, oxamyl, Paecilomyces fumosoroseus, phosmet, pirimicarb, pirimiphos M, pyrethrum, pyridaben, pyriproxyfen, piperonyl butoxide, spinosad, spironesifen, spirotetramat, spinetoran, spirodiclofen, tau-fluvalinate, tebufenozide, tebufenpyrad, teflubenzuron, tefluthrin, thiacloprid, triflumuron, zeta-cypermethrin, (1R-cis)-[5-(phenylmethyl)-3-furanyl]-methyl-3-[(dihydro-2-oxo-3(2H)-furanylidene) methyl] -2,2-dimethyl-cyclo-propanecarboxylate, (3-phenoxyphenyl)-methyl-2,2,3,3-tetramethyl-cyclopropanecarboxylate, 1-[(2-chloro-5-thiazolyl)methyl]-5-triazine 2-(1H)-imine, 2-(2-chloro-6-fluorophenyl)-4-[4-(1,1-dimethylethyl)-phenyl]-4,5-dihydro-oxazol, 2-(acetyloxy)-3-dodecyl-1,4-naph-thalenedione, 2-chloro-N-[[[4-(1-phenylethoxy)-phenyl]-amino]-carbonyl]-benzamide, 2-chloro-N-[[[4-(2,2-dichloro-1,1-difluoroethoxy)-phenyl] -amino] -carbonyl] -benzamide, 3-methylphenyl-propylcarbamate, 4-[4-(4-ethoxyphenyl)-4-methylpentyl]-1-fluoro-2-phenoxy-benzene, 4-chloro-2-(1,1-dimethylethyl)-5-[[2-(2,6-dimethyl-4-phenoxyphenoxy)ethyl]thio]-3-(2H)-pyridazinone, 4-chloro-2-(2-chloro-2-methylpropyl)-5-[(6-iodo-3-pyridinyl)(2-chloro-2-methylpropyl)-5-[(6-iodo-3-pyridinyl)-methoxy]-3-(2H)pyridazinone, 4-chloro-5-[(6-chloro-3-pyridinyl)methoxy]-2-(3,4-dichlorophenyl)-3(2H)pyridazinone, Bacillus thuringiensis strain EG-2348, [2-benzoyl-1-(1,1-dimethylethyl)-hydrazine] benzoic acid, 2,2-dimethyl-3-(2,4-dichlorophenyl)-2-oxo-1-oxaspiro [4.5]-dec-3-en-4-yl butanoate, [3-[(6-chloro-3-pyridinyl)-methyl]-2-thiazolidinylidene]-cyanamide, dihydro-2-(nitro-methylene)-2H-1,3-thiazine-3(4H)-carboxaldehyde, ethyl[2-[[1,6-dihydro-6-oxo-1-(phenylmethyl)-4-pyrid-azinyl]oxy]ethyl]-carbamate, N-(3,4,4-trifluoro-1-oxo-3-butenyl)-glycine, N-(4-chlorophenyl)-3-[4-(difluoro-methoxy)-phenyl]-4,5-dihydro-4-phenyl-1H-pyrazole-1-carboxamide, N-[(2-chloro-5-thiazolyl)-methyl]-N'-methyl-N"-nitro-guanidine, N-methyl-N'-(1-methyl-2-propenyl)-1,2-hydrazinedicarbo-thioamide, N-methyl-N'-2-propenyl-1,2-hydrazinedicarbo-thioamide, O,O-diethyl[2-(dipropylamino)-2-oxoethyl]-ethyl-phosphoro-amidothioate.

Examples of herbicides that can be added to the compositions containing one or more compounds having general formula (I) are the following: acetochlor, acifluorfen, aclonifen, AKH-7088 ({methyl (E,Z)-[[[1-[2-chloro-4-(trifluoromethyl)phenoxy]-2-nitrophenyl]-2-methoxyethylidene]amino]acetate}), alachlor, alloxydim, ametryn, amicarbazone, amidosulfuron, amitrole, anilofos, asulam, atrazine, azafenidin, azimsulfuron, aziprotryne, BAY MKH 6561 (methyl 2-({[(4-methyl-5-oxo-3-propoxy-4,5-dihydro-1H-1,2,4-triazol-1-yl)carbonyl] amino}sulfonyl)benzoate sodium salt), beflubutamid, benazolin, benfluralin, benfuresate, bensulfuron, bensulide, bentazone, benzfendizone, benzobicyclon, benzofenap, benzthiazuron, bifenox, bilanafos, bispyribac-sodium, bromacil, bromobutide, bromofenoxim, bromoxynil, butachlor, butafenacil, butamifos, butenachlor, butralin, butroxydim, butylate, cafenstrole, carbetamide, carfentrazone-ethyl, chlomethoxyfen, chloramben, chlorbromuron, chlorbufam, chlorflurenol, chloridazon, chlorimuron, chlornitrofen, chlorotoluron, chloroxuron, chlorpropham, chlorsulfuron, chlorthal, chlorthiamid, cinidon ethyl, cinmethylin, cinosulfuron, clethodim, clodinafop, clomazone, clomeprop, clopyralid, cloransulam-methyl, cumyluron (JC-940), cyanazine, cycloate, cyclosulfamuron, cycloxydim, cyhalofop-butyl, 2,4-D, 2,4-DB, daimuron, dalapon, desmedipham, desmetryn, dicamba, dichlobenil, dichlorprop, dichlorprop-P, diclofop, diclosulam, diethatyl, difenoxuron, difenzoquat, diflufenican, diflufenzopyr, dimefuron, dimepiperate, dimethachlor, dimethametryn, dimethenamid, dinitramine, dinoseb, dinoseb acetate, dinoterb, diphenamid, dipropetryn, diquat, dithiopyr, 1-diuron, eglinazine, endothal, EPTC, esprocarb, ethalfluralin, ethametsulfuron-methyl, ethidimuron, ethiozin (SMY 1500), ethofumesate, ethox-yfen-ethyl (HC-252), ethoxysulfuron, etobenzanid (HW 52), fenoxaprop, fenoxaprop-P, fentra-zamide, fenuron, flamprop, flamprop-M, flazasulfuron, florasulam, fluazifop, fluazifop-P, fluazolate (JV 485), flucarbazone-sodium, fluchloralin, flufenacet, flufenpyr ethyl, flumetsulam, flumiclorac-pentyl, flumioxazin, flumipropin, fluometuron, fluoroglycofen, fluoronitrofen, flupoxam, flupropanate, flupyrsulfuron, flurenol, fluridone, flurochloridone, fluroxypyr, flurtamone, fluthiacet-methyl, fomesafen, foramsulfuron, fosamine, furyloxyfen, glufosinate, glyphosate, halosulfuron-methyl, haloxyfop, haloxyfop-P-methyl, hexazinone, imazamethabenz, imazamox, imazapic, imazapyr, imazaquin, imazethapyr, imazosulfuron, indanofan, iodosulfuron, ioxynil, isopropalin, isoproturon, isouron, isoxaben, isoxachlortole, isoxaflutole, isoxapyrifop, KPP-421, lactofen, lenacil, linuron, LS830556 [[[2-methyl(methylsulfonyl)amino]-2-oxoethyl]amino]methylphosphonic acid, MCPA 2-methyl-4-chlorophenoxyacetic acid, MCPA-thioethyl, MCPB (4-(4-chloro-2-methylphenoxy)butanoic acid), mecoprop, mecoprop-P, mefenacet, mesosulfuron, mesotrione, metamitron, metazachlor, methabenzthiazuron, methazole, methoprotryne, methyldymron, metobenzuron, metobromuron, metolachlor, S-metolachlor, metosulam, metoxuron, metribuzin, metsulfuron, molinate, monalide, monolinuron, naproanilide, napropamide, naptalam, NC-330 (methyl 5-[(4,6-dimethylpyrimidin-2-yl)carbamoylsulfamoyl]1-pyridin-2-yl pyrazol-4-carboxylate), neburon, nicosulfuron, nipyraclofen, norflurazon, orbencarb, oryzalin, oxadiargyl, oxadiazon, oxasulfuron, oxaziclomefone, oxyfluorfen, paraquat, pebulate, pendimethalin, penoxsulam, pentanochlor, pentoxazone, pethoxamid, phenmedipham, picloram, picolinafen, piperophos, pretilachlor, primisulfuron, prodiamine, profluazol, proglinazine, prometon, prometryne, propachlor, propanyl, propaquizafop, propazine, propham, propisochlor, propyzamide, prosulfocarb, prosulfuron, pyraclonil, pyraflufen-ethyl, pyrazogyl (HSA-961), pyrazolynate, pyrazosulfuron, pyrazoxyfen, pyribenzoxim, pyributicarb, pyridafol, pyridate, pyriftalid, pyriminobac-methyl, pyrithiobac-sodium, pyroxasulfone quinclorac, quinmerac, quizalofop, quizalofop-P, rimsulfuron, sethoxydim, siduron, simazine, simetryn, sulcotrione, sulfentrazone, sulfometuron-methyl, sulfosulfuron, 2,3,6-TBA, TCA-sodium, tebutam, tebuthiuron, tepraloxydim, terbacil, terbumeton, terbuthyl-azine, terbutryn, thenylchlor, thiazafluron, thiazopyr, thidiazimin, thifensulfuron-methyl, thiobencarb, tiocarbazil, tioclorim, tralkoxydim, tri-allate, triasulfuron, triaziflam, tribenuron, triclopyr, trietazine, trifloxysulfuron, trifluralin, triflusulfuron-methyl, tritosulfuron, UBI-C4874 (quizalofop-P), vernolate.

Examples of fungicides that can be added to the compositions containing one or more compounds having general formula (I) are the following: acibenzolar, ametoctradin, amisulbrom, ampropylfos, anilazine, azaconazole, azoxystrobin, benalaxyl, benalaxyl-M, benomyl, benthiavalicarb, bitertanol, bixafen, blasticidin-S, boscalid, bromuconazole, bupirimate, buthiobate, captafol, captan, carbendazim, carboxin, carpropamid, chinomethionat, chloroneb, chlorothalonil, chlozolinate, cyazofamid, cyflufenamid, cymoxanil, cyproconazole, cyprodinil, debacarb, dichlofluanid, dichlone, diclobutrazol, diclomezine, dicloran, diclocymet, diethofencarb, difenoconazole, diflumetorim, dimethirimol, dimethomorph, dimoxystrobin, diniconazole, dinocap, dipyrithione, ditalimfos, dithianon, dodemorph, dodine, edifenphos, epoxiconazole, etaconazole, ethaboxam, ethirimol, ethoxyquin, etridiazole, famoxadone, fenamidone, fenaminosulf, fenapanil, fenarimol, fenbuconazole, fenfuram, fenhexamid, fenoxanil, fenpiclonil, fenpropidin, fenpropimorph, fenpyrazamine, fentin, ferbam, ferimzone, fluazinam, fludioxonil, fluindapyr ((*RS*)-3-(difluoromethyl)-*N*-(7-fluoro-2,3-dihydro-1,1,3-trimethyl-1*H*-inden-4-yl)-1-methyl-1*H-*pyrazole-4-carboxamide), flumetover, flumorph, fluopicolide, fluopyram, fluoroimide, fluotri-mazole, fluoxastrobin, fluquinconazole, flusilazole, flusulfamide, flutianil, flutolanil, flutriafol, fluxapyroxad, folpet, fosetyl-aluminium, fuberidazole, furalaxyl, furametpyr, furconazole, furconazole-cis, guazatine, hexaconazole, hymexazol, hydroxyquinoline sulfate, imazalil, imibenconazole, iminoctadine, ipconazole, iprobenfos, iprodione, isoprothiolane, iprovalicarb, isopyrazam, isotianil, kasugamycin, kresoxim-methyl, mancopper, mancozeb, mandipropamid, maneb, mebenil, mepanipyrim, mepronil, meptyldinocap, metalaxyl, metalaxyl-M, metconazole, methfuroxam, metiram, metominostrobin, metrafenone, metsulfovax, myclobutanil, natamycin, nicobifen, nitrothal-isopropyl, nuarimol, ofurace, orysastrobin, oxadixyl, oxpoconazole, oxycarboxin, pefurazoate, penconazole, pencycuron, penflufen, pentachlorofenol and its salts, penthiopyrad, phthalide, picoxystrobin, piperalin, Bordeaux mixture, polyoxins, probenazole, prochloraz, procymidone, propamocarb, propiconazole, propineb, proquinazid, prothiocarb, prothioconazole, pyracarbolid, pyraclostrobin, pyrametostrobin, pyraoxystrobin, pyrazophos, pyribencarb, pyrifenox, pyrimethanil, pyriofenone, pyroquilon, pyroxyfur, quinacetol, quinazamid, quinconazole, quinoxyfen, quintozene, rabenzazole, copper hydroxide, copper oxychloride, copper (I) oxide, copper sulfate, sedaxane, silthiofam, simeconazole, spiroxamine, streptomycin, tebuconazole, tebufloquin, tetraconazole, thiabendazole, thiadifluor, thicyofen, thifluzamide, thiophanate, thiophanate-methyl, thiram, tiadinil, tioxymid, tolclofos-methyl, tolylfluanid, triadimefon, triadimenol, triarimol, triazbutil, triazoxide, tricyclazole, tridemorf, trifloxystrobin, triflumizole, triforine, triticonazole, uniconazole, uniconazole-P, validamycin, valifenalate, vinclozolin, zineb, ziram, sulfur, zoxamide.

Examples of bactericides that can be added to the compositions containing one or more compounds having general formula (I) are the following: bronopol, dichlorophen, nitrapyrina, nickel dimethyldithiocarbamate, kasugamycin, octhilinone, furancarboxylic acid, probenazole, streptomycin, tecloftalam, copper hydroxide, copper oxychloride, copper (I) oxide, copper sulfate, copper salicylate.

Examples of fertilizers and biostimulants that can be added to the compositions containing one or more compounds having general formula (I) are the following: mixtures of amino acids and/or oligopeptides of an animal and/or vegetable origin, 4-thiazolidinecarboxylic acid, 4-acetylthiazolidine-carboxylic acid, ectoin, phytosterols.

A further object of the present invention therefore relates to agronomic compositions comprising at least one compound having Formula (I) and at least a second active ingredient selected from insecticides, acaricides, nematocides other than those having Formula (I), herbicides, fungicides, bactericides, fertilizers and biostimulants.

The compositions object of the present invention are capable of exerting a nematocidal action that can be of a curative and/or preventive nature and generally have an extremely low or zero phytotoxicity with respect to the crops treated.

A further object of the present invention therefore relates to the use of compositions comprising at least one compound having Formula (I) for the control of nematodes.

When the compositions comprise a compound having Formula (I) and at least a further known active ingredient, the weight ratios in the above compositions, between the compound having Formula (I) and the further known active ingredients, range according to the compounds selected and can normally be within the range of 1:100 to 100:1, preferably from 1:10 to 10:1.

The total concentration of active components in the above compositions can vary within a wide range; they generally range from 1% to 99% by weight with respect to the total weight of the composition, preferably from 5 to 90% by weight with respect to the total weight of the composition.

The compounds having Formula (I) or the compositions containing them can be applied to the crop via the leaves, or to the soil by means of fertigation, or incorporation into the ground, or through seed care.

A further object of the present invention also relates to a method for the control of nematodes in cultivated areas, which consists in applying effective and non-phytotoxic doses of compositions comprising compounds having Formula (I) and, optionally, one or more known active ingredients compatible therewith, on any part of the plant to be protected.

The compounds according to the present invention can be applied from one to seven days before transplanting the plant to be protected or directly with the already cultivating crop, without providing any symptom of phytotoxicity. This latter aspect makes them particularly interesting from an agronomical point of view, as they can be used at any time of growth of the plant without damaging it.

The quantity of compound to be applied for obtaining the desired effect can vary in relation to different factors such as, for example, the compound used, the crop to be protected, the degree of infestation, the climatic conditions, the characteristics of the soil, the method of application, etc. Doses of compound having Formula (I) ranging from 100 g to 10,000 g per hectare of agricultural crop or, in the case of compositions comprising other known active ingredients, overall doses of active ingredients ranging from 100 g to 20,000 g per hectare of agricultural crop, generally provide a sufficient control.

Doses of compound having Formula (I) ranging from 500 g to 800 g per hectare of agricultural crop are preferably used.

The following illustrative and non-limiting examples are provided for a better understanding of the invention.

### EXAMPLE 1

### Preparation of 2-(3,4,4-trifluoro-3-butenylsulfonyl)-5-methyl-1,3,4-thiadiazole [Compound N°2].

### a) Preparation of 2-(3,4,4-trifluoro-3-butenylthio)-5-methyl-1,3,4-thiadiazole [thioether having general formula (II)]

1.1 ml (7.9 mmoles) of triethylamine were added at room temperature to a suspension under nitrogen of 0.7 g (5.3 mmoles) of 5-methyl-1,3,4-thiadiazole-2-thiol in 15 ml of chloroform. After 30 minutes, 1 g (5.3 mmoles) of 4-bromo-1,1,2-trifluorobutene were then added and the reaction mixture was heated to 50°C for 2 hours.

After control in GC-MS and LC-MS, the mixture was diluted with water and dichloromethane, and the phases were then separated; the aqueous phase was re-extracted twice with dichloroethane. The organic phases were joined and washed with water and a saturated solution of sodium chloride.

After anhydrification on sodium sulfate, filtration and evaporation of the solvent at reduced pressure, 1.03 g of the desired product were obtained (4.3 mmoles), as a white solid. Yield 82% LC-MS [M+H] = 239.

### b) Preparation of 2-(3,4,4-trifluoro-3-butenyl-sulfonyl)-5-methyl-1,3,4-thiadiazole [Compound N°2].

2.8 g (12.9 mmoles) of 4-chloroperbenzoic acid at 77% were added to 1.03 g (4.3 mmoles) of 2-(3,4,4-trifluoro-3-butenylthio)-5-methyl-1,3,4-thiadiazole, dissolved in 30 ml of chloroform, maintaining a temperature of about 4-5°C with an ice bath. The mixture was then left under magnetic stirring at room temperature for a night.

After control in LC-MS, the mixture was diluted with water, and the phases were then separated; the aqueous phase was re-extracted twice with dichloromethane. The organic phases were joined and washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 5.1 g of yellow oil (15.8 mmoles) were obtained. The raw product thus obtained was treated with ethyl ether, filtered and dried in air, obtaining 0.9 g (33 mmoles) of the desired product as a white solid. Yield 77% LC-MS [M+H] = 272.

### EXAMPLE 2

### Preparation of 2-(3,4,4-trifluoro-3-butenylsulfinyl)-5-methyl-1,3,4-thiadiazole [Compound N°1]

2.95 g (9.17 mmoles) of 4-chloroperbenzoic acid at 77% were added to 2 g (8.33 mmoles) of 2-(3,4,4-trifluoro-3-butenylthio)-5-methyl-1,3,4-thiadiazole, dissolved in 60 ml of chloroform, maintaining a temperature of about 4-5°C with an ice bath. The mixture was then left under magnetic stirring at room temperature for a night.

After control in LC-MS, the mixture was diluted with water, and the phases were then separated; the aqueous phase was re-extracted twice with dichloromethane. The organic phases were joined and washed with an aqueous solution at 5% of NaHSO₃, a saturated solution of NaHCO₃, water and a saturated solution of NaCl.

After anhydrification on sodium sulfate, filtration and evaporation of the solvent at reduced pressure, 1.9 g of the desired product (15.9 mmoles) were obtained as a yellow oil. The raw product thus obtained was treated with ethyl ether, filtered and dried in air, obtaining 4.2 g (13.7 mmoles) of the desired product as a yellow oil.
Yield 89.1% LC-MS [M+H] = 256.

### EXAMPLE 3

### Preparation of 2-(3,4,4-trifluoro-3-butenylsulfonyl)-5-chloro-1,3,4-thiadiazole [Compound N°22].

### a) Preparation of 2-(3,4,4-trifluoro-3-butenylthio)-5-amino-1,3,4-thiadiazole [thioether having general formula (II)]

20.65 ml (41.3 mmoles) of an aqueous solution of sodium hydroxide 2M were added at room temperature to a suspension under nitrogen of 5 g (37.5 mmoles) of 5-amino-1,3,4-thiadiazole-2-thiol in 27 ml of ethanol. After 30 minutes, 4.32 ml (37.54 mmoles) of 4-bromo-1,1,2-trifluorobutene were then added and the reaction mixture was heated to reflux temperature for 4 hours.

After control in GC-MS and LC-MS, the mixture was concentrated, diluted with water and ethyl acetate, and the phases were then separated; the aqueous phase was re-extracted twice with ethyl acetate. The organic phases were joined and washed with a solution of sodium hydroxide at 2%, then with water and finally with a saturated solution of sodium chloride.

After anhydrification on sodium sulfate, filtration and evaporation of the solvent at reduced pressure, 7.88 g of the desired product were obtained (4.3 mmoles), as a white solid. Yield 88% LC-MS [M+H] = 242.

### b) Preparation of 2-(3,4,4-trifluoro-3-butenylthio)-5-chloro-1,3,4-thiadiazole [thioether having general formula (II)]

6 g (24.9 mmoles) of 2-(3,4,4-trifluoro-3-butenylthio)-5-amino-1,3,4-thiadiazole were added to a solution of 0.6 g of copper powder in 60 ml of hydrochloric acid, under nitrogen and at 0°C.

A solution of 1.89 g of sodium nitrite in 17 ml of water was slowly added dropwise to the suspension thus obtained.

The mixture was left under magnetic stirring at room temperature for 4 hours.

After control in GC-MS and LC-MS, the mixture was diluted with water and chloroform, and the phases were then separated; the aqueous phase was re-extracted twice with chloroform. The organic phases were joined and washed with water and a saturated solution of sodium chloride. After anhydrification on sodium sulfate, filtration and evaporation of the solvent at reduced pressure, 4.08 g of the desired product were obtained (15.6 mmoles), as a yellow oil.
Yield 62.7% LC-MS [M+H] = 260.

### c) Preparation of 2-(3,4,4-trifluoro-3-butenyl-sulfonyl)-5-chloro-1,3,4-thiadiazole [Compound N°22].

Operating analogously to Example 1b) starting from 3.08 g (11.8 mmoles) of 2-(3,4,4-trifluoro-3-butenylthio)-5-chloro-1,3,4-thiadiazole, 3.02 g (10.4 mmoles) of the desired product were obtained, after processing of the reaction.
Yield 87.3% LC-MS [M+H] = 292

### EXAMPLE 4

### Determination of the nematocidal activity against Meloidogyne ssp. (application 7 days before transplantation)

The tests, aimed at testing the nematocidal activity of the product under consideration, were carried out using inocula taken from a breeding of *Meloidogyne spp.* maintained on tomato and tobacco plants in pots and grown in a greenhouse.

In order to perform the experiments, portions of roots having a large number of galls, and ground, in which larvae are present, starting from the second stage of age, were collected from the infested pots.

New pots having a diameter of 15 cm were half-filled with sterile earth. The portions of infested roots, previously cleaned, were positioned on the same in order to correctly evaluate the degree of infestation and ensure that each pot contained the same nematic charge. 200-300 g of infested earth were then added and subsequently covered with a thin layer of sterile earth.

The treatment was effected by pouring, onto the surface of the ground, 100 ml of solution in which the product to be tested was dissolved.

Tomato seedlings at a stage of two-three true leaves were transplanted into the pots thus prepared. Different tomato cultivars were adopted, having a different sensitivity to the parasite and with different growth rates. In particular, an ornamental variety (cv Microtom) was used for evaluating the final production, whose seedlings have reduced dimensions and are capable of reaching maturity of the fruit in pots and under greenhouse conditions, in about two months.

The evaluation of the product under consideration was effected by treating the pots 7 days before transplantation (according to what is indicated in the label of the commercial reference product NIMITZ® (Fluensulfone)).

The product was evaluated considering the possible phytotoxic effects that can jeopardize the development of the plant (a zero-ten scale was used wherein: zero = no symptoms, 10 = plant destroyed) and the effectiveness with respect to nematodes.

The capacity of limiting the parasite was detected, at a distance of 30 and 60 days after transplanting, considering the development of the root system (wherein 100% is the development reached by the comparative healthy root) and the presence of galls on the roots. The presence of galls in the roots was estimated using the infestation scale proposed by Bridge and Page, in which the value zero corresponds to 0% of affected root and the value of 10 corresponds to 100% of infested root.

Table 2 indicates the results relating to the effectiveness of Compounds 1,2 and 22 and Compounds CR1, CR2, CR3 on tomatoes at a dosage of 4,000 g/hectare, effecting the assessment 60 days after the transplantation.

**Table 2: Pre-transplantation treatment (7 days before transplantation):**

| Compound | Gall index (scale 0-10) | Height of plant (cm) | Root development |
|---|---|---|---|
| Infested blank | 8.0 | 61 | 40 |
| Nr 2 | 1.0 | 76 | 95 |
| Nr 1 | 1.0 | 75 | 95 |
| Nr 22 | 1.5 | 73 | 90 |
| CR1 | 2.0 | 71 | 70 |
| CR2 | 2.0 | 60 | 70 |
| CR3 | 1.5 | 70 | 75 |

| | | | |
|---|---|---|---|
| **CR1**=5-chloro-2-(3,4,4-trifluoro-3-butenylsulfonyl)-thiazole (indicated in WO01/02378, page 17, example 3), also known as the commercial product NIMITZ® (Fluensulfone); **CR2**=2-(3,4,4-trifluoro-3-butenylthio)-5-methyl-1,3,4-thiadiazole (indicated in EP410551, page 5, compound N°14); **CR3=**2-(4,4-difluoro-3-butenylsulfonyl)-5-methyl-1,3,4-thiadiazole (indicated in WO95/24403, page 52, compound N°XIII.42). | | | |

As can be observed in Table 2, compounds Nr.1, 2 and 22 of the present invention proved to be more active in the control of nematodes with respect to the products previously described in literature (gall index lower or comparable to that of the reference compounds). It can also be noted that the application of the above compounds led to a higher growth of the plant and/or greater development of the root than that observed with the previously known compounds, representing a significant advantage of the products of the present invention.

### EXAMPLE 5

### Determination of the nematocidal activity against Meloidogyne ssp. (application at the moment of transplantation)

The same experimental procedure as Example 4 was adopted for evaluating the nematocidal activity of the products under examination, except for the fact that this evaluation was effected by applying the product at the moment of transplantation.

Table 3 indicates the results relating to the effectiveness of compounds 9, 10 and the reference compound CR1 on tomatoes at a dose of 4,000 g/hectare effecting the survey 60 days after transplantation.

**Table 3. Treatment at the moment of transplantation (1 treatment at the moment of transplantation):**

| Compound | Gall index (scale 0-10) | Weight of plant (g) | Root development |
|---|---|---|---|
| Infested blank | 4.0 | 70 | 50 |
| Nr 9 | 2.0 | 116 | 70 |
| Nr 10 | 2.0 | 88 | 75 |
| CR1 | 1.5 | 62 | 60 |

In this test with a single treatment at the moment of transplantation, although compounds Nr 9 and Nr 10 provided a slightly lower effectiveness than that of the reference product, it can be observed that the weight of the plant and/or the root development of the plants treated with these products are much higher compared with the same parameters as the reference compound. In particular, it can be observed that for compound Nr 9, the weight of the plant after treatment reaches almost 120 g, i.e. double the weight of the plant treated with the compound CR1.

### EXAMPLE 6

### Determination of the nematocidal activity against Meloidogyne ssp. (application with already cultivating crop)

The same experimental procedure as Example 4 was adopted for evaluating the nematocidal activity of the products under examination, except for the fact that this evaluation was effected by applying the product during the development of the seedlings (according to what is indicated in the label of the commercial reference product VYDATE® (Oxamil)).

Table 4 indicates the results relating to the effectiveness of compounds 1, 2, 6 and the compound CR1 on tomatoes at a dose of 4,000 g/hectare effecting the survey 60 days after transplantation.

**Table 4. Treatment with already cultivating crop (repeated treatment after transplantation):**

| Compound | Gall index (scale 0-10) | Weight of plant (g) | Root development |
|---|---|---|---|
| Infested blank | 8.5 | 17 | 22 |
| Nr 2 | 0.0 | 33 | 100 |
| Nr 1 | 1.0 | 36 | 100 |
| Nr 6 | 2.0 | 40 | 95 |
| CR1 | 1.5 | 25 | 32 |
| VYDATE® | 2.0 | 33 | 90 |

Compounds Nr 1 and Nr 2 proved to be particularly effective in limiting the very strong attack of nematodes (gall index 8.5).

Furthermore, as compound Nr 6 showed a slightly lower nematocidal activity with respect to the reference compound CR1 (also known as NIMITZ®) but in any case comparable to that of the commercial product VYDATE®, it can be easily noted that the root of the plant treated with this compound is much more developed with respect to that treated with CR1, indicating that the plant itself is in excellent health in spite of the attack of nematodes.

Table 5 indicates the results relating to phytotoxicity on tomatoes applying compounds 1, 2, 6, 9, and CR1 with already cultivating crop:

**Table 5**

| Compound Nr | Phytotoxicity (scale 0-10) |
|---|---|
| 1 | 0 |
| 2 | 0 |
| 6 | 0 |
| 9 | 0 |
| CR1 | 3 |

These results confirm what is indicated in the label of the reference compound CR1 (NIMITZ®), i.e. the impossibility of using this product with already cultivating crop.

As can be seen from the experimental data indicated above, on the contrary, the compounds according to the invention can be used at any moment of cultivation, i.e. application 7 days before transplantation, at the moment of transplantation and with already cultivating crop, without damaging the treated plant.

## Claims

1. Heterocyclic trifluoroalkenyl compounds having formula (I)
Het-S(O)ₙ-(CH₂)ₘ-CF=CF₂ (I)
wherein:
- Het represents an aromatic heterocyclic group, substituted, selected from: 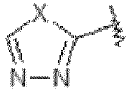
- X represents a sulfur atom;
- n represents 1 or 2;
- m represents 1 or 2,
wherein the substituents of the heterocycle Het are selected from halogen; a C₁-C₆ alkyl; a C₁-C₆ haloalkyl; a C₃-C₆ cycloalkyl; formyl; optionally substituted aryl; benzyl; an aromatic penta- or hexa-atomic heterocyclic group, optionally benzocondensed or heterobicyclic, containing at least one heteroatom selected from oxygen, sulfur, nitrogen or an optionally substituted N-oxide, preferably selected from oxygen, sulfur or nitrogen.

2. The compounds according to claim 1, wherein
- Het, substituted, represents: 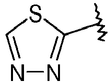
- the substituent of the heterocyclic group is a C1-C6 alkyl group;
- m is equal to 2.

3. Compounds according to claim 1, wherein Het, n and m have the following meanings:
| Nr | Het | n | m |
|---|---|---|---|
| 1 | 5-methyl-1,3,4-thiadiazole | 1 | 2 |
| 2 | 5-methyl-1,3,4-thiadiazole | 2 | 2 |
| 3 | 5-ethyl-1,3,4-thiadiazole | 1 | 2 |
| 4 | 5-ethyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 7 | 5-propyl-1,3,4-thiadiazole | 1 | 2 |
| 8 | 5-propyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |
| 11 | 5-cyclopropyl-1,3,4-thiadiazole | 1 | 2 |
| 12 | 5-cyclopropyl-1,3,4-thiadiazole | 2 | 2 |
| 13 | 5-cyclohexyl-1,3,4-thiadiazole | 1 | 2 |
| 14 | 5-cyclohexyl-1,3,4-thiadiazole | 2 | 2 |
| 15 | 5-trifluoromethyl-1,3,4-thiadiazole | 1 | 2 |
| 16 | 5-trifluoromethyl-1,3,4-thiadiazole | 2 | 2 |
| 17 | 5-difluoromethyl-1,3,4-thiadiazole | 1 | 2 |
| 18 | 5-difluoromethyl-1,3,4-thiadiazole | 2 | 2 |
| 19 | 5-phenyl-1,3,4-thiadiazole | 1 | 2 |
| 20 | 5-phenyl-1,3,4-thiadiazole | 2 | 2 |
| 21 | 5-chloro-1,3,4-thiadiazole | 1 | 2 |
| 22 | 5-chloro-1,3,4-thiadiazole | 2 | 2 |
| 23 | 5-bromo-1,3,4-thiadiazole | 1 | 2 |
| 24 | 5-bromo-1,3,4-thiadiazole | 2 | 2 |
| 25 | 5-thienyl-1,3,4-thiadiazole | 1 | 2 |
| 26 | 5-thienyl-1,3,4-thiadiazole | 2 | 2 |
| 27 | 5-isopentyl-1,3,4-thiadiazole | 1 | 2 |
| 28 | 5-isopentyl-1,3,4-thiadiazole | 2 | 2 |
| 29 | 5-(2,2-dimethylpropyl)-1,3,4-thiadiazole | 1 | 2 |
| 30 | 5-(2,2-dimethylpropyl)-1,3,4-thiadiazole | 2 | 2 |
| 31 | 5-(2-methylbutyl)-1,3,4-thiadiazole | 1 | 2 |
| 32 | 5-(2-methylbutyl)-1,3,4-thiadiazole | 2 | 2 |
| 33 | 5-methylcyclohexyl-1,3,4-thiadiazole | 1 | 2 |
| 34 | 5-methylcyclohexyl-1,3,4-thiadiazole | 2 | 2 |
| 35 | 5-methylcyclopropyl-1,3,4-thiadiazole | 1 | 2 |
| 36 | 5-methylcyclopropyl-1,3,4-thiadiazole | 2 | 2 |

4. The compounds according to claim 1, wherein Het, n and m have the following meanings:
| Nr | Het | n | m |
|---|---|---|---|
| 1 | 5-methyl-1,3,4-thiadiazole | 1 | 2 |
| 2 | 5-methyl-1,3,4-thiadiazole | 2 | 2 |
| 3 | 5-ethyl-1,3,4-thiadiazole | 1 | 2 |
| 4 | 5-ethyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |

5. The compounds according to any of claims 1-5, wherein n is equal to 1, in racemic form, isomerically pure, or mixtures thereof.

6. Use of heterocyclic trifluoroalkenyl compounds having formula (I)
Het-S(O)ₙ-(CH₂)ₘ-CF=CF₂ (I)
wherein:
- Het represents an aromatic heterocyclic group, optionally substituted, selected from: 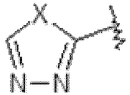
- X represents a sulfur atom;
- n represents 1 or 2;
- m represents 1 or 2
for the control of nematodes.

7. Use of heterocyclic trifluoroalkenyl compounds having formula (I) according any of the claims from 2 to 6 for the control of nematodes, preferably for the control of Pratylenchus spp, Globodera spp, Heterodera spp, Meloidogyne spp, Aphelenchoides spp, Radopholus Similis, Ditylenchus Dipsaci, Tylenchulus Semipenetrans, Longidorus spp, Xiphinema spp, Trichodorus spp, Bursaphelenchus spp, of a curative and/or preventive nature.

8. Agronomic compositions comprising one or more compounds having formula (I) according to one or more of the claims from 1 to 6, in combination with a solvent and/or solid, liquid or liquefied diluent, optionally one or more surfactants and other agronomically acceptable co-formulants.

9. The agronomic compositions according to claim 8, wherein the concentration of active compound having formula (I) ranges from 0.1 to 90% by weight with respect to the total weight of the composition, preferably from 0.5 to 90% by weight.

10. The agronomic compositions according to one or more of the claims from 8 to 9, comprising at least one compound having formula (I) and at least a second active ingredient selected from insecticides, acaricides, nematocides other than those having formula (I), herbicides, fungicides, bactericides, fertilizers and biostimulants.

11. The agronomic compositions according to claim 10, wherein the weight ratio between the compound having formula (I) and the further active ingredients ranges from 1:100 to 100:1, preferably from 1:10 to 10:1.

12. The agronomic compositions according to claim 10, wherein the concentration of active ingredient ranges from 0.5 to 90% by weight with respect to the total weight of the composition, preferably from 5 to 90% by weight.

13. Use of the agronomic compositions according to one or more of the claims from 8 to 12, for the control of nematodes.

14. Use of the agronomic compositions according to claim 13 by application of the composition to the crop via the leaves, or to the soil by means of fertigation, or incorporation into the ground, or through seed care.

15. A method for the control of nematodes in cultivated areas, which consists in applying, to any part of the plants to be protected, effective and non-phytotoxic dosages of agronomic compositions according to any of the previous claims from 8 to 12, comprising at least one compound having formula (I), and, optionally, one or more further known active ingredients compatible therewith.

## Patentansprüche

1. Heterozyklische Trifluoralkenyl-Verbindungen der Formel (I)
Het-S(O)ₙ-(CH₂)ₘ-CF=CF₂ (I)
wobei:
- Het für eine aromatische heterozyklische Gruppe, substituiert, steht, die aus Folgenden ausgewählt ist: 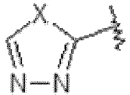
- X für ein Schwefelatom steht;
- n für 1 oder 2 steht;
- m für 1 oder 2 steht,
wobei die Substituenten des Heterocyclyls Het aus Halogen; einem C₁-C₆-Alkyl; einem C₁-C₆-Halogenalkyl; einem C₃-C₆-Cycloalkyl; Formyl; gegebenenfalls substituiertem Aryl; Benzyl; einer aromatischen fünfwertigen oder sechswertigen heterozyklischen Gruppe, die gegebenenfalls benzokondensiert oder heterobizyklisch ist, die mindestens ein Heteroatom enthält, das aus Sauerstoff, Schwefel, Stickstoff oder einem gegebenenfalls substituierten N-Oxid, vorzugsweise aus Sauerstoff, Schwefel oder Stickstoff ausgewählt ist.

2. Verbindungen nach Anspruch 1, wobei:
- Het, substituiert, für Folgendes steht: 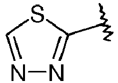
- der Substituent der heterozyklischen Gruppe eine C₁-C₆-Alkylgruppe ist;
- m = 2 ist.

3. Verbindungen nach Anspruch 1, wobei Het, n und m die folgenden Bedeutungen aufweisen:
| Nr | Het | n | m |
|---|---|---|---|
| 1 | 5-Methyl-1,3,4-thiadiazol | 1 | 2 |
| 2 | 5-Methyl-1,3,4-thiadiazol | 2 | 2 |
| 3 | 5-Ethyl-1,3,4-thiadiazol | 1 | 2 |
| 4 | 5-Ethyl-1,3,4-thiadiazol | 2 | 2 |
| 5 | 5-Isopropyl-1,3,4-thiadiazol | 1 | 2 |
| 6 | 5-Isopropyl-1,3,4-thiadiazol | 2 | 2 |
| 7 | 5-Propyl-1,3,4-thiadiazol | 1 | 2 |
| 8 | 5-Propyl-1,3,4-thiadiazol | 2 | 2 |
| 9 | 5-Isobutyl-1,3,4-thiadiazol | 1 | 2 |
| 10 | 5-Isobutyl-1,3,4-thiadiazol | 2 | 2 |
| 11 | 5-Cyclopropyl-1,3,4-thiadiazol | 1 | 2 |
| 12 | 5-Cyclopropyl-1,3,4-thiadiazol | 2 | 2 |
| 13 | 5-Cyclohexyl-1,3,4-thiadiazol | 1 | 2 |
| 14 | 5-Cyclohexyl-1,3,4-thiadiazol | 2 | 2 |
| 15 | 5-Trifluormethyl-1,3,4-thiadiazol | 1 | 2 |
| 16 | 5-Trifluormethyl-1,3,4-thiadiazol | 2 | 2 |
| 17 | 5-Difluormethyl-1,3,4-thiadiazol | 1 | 2 |
| 18 | 5-Difluormethyl-1,3,4-thiadiazol | 2 | 2 |
| 19 | 5-Phenyl-1,3,4-thiadiazol | 1 | 2 |
| 20 | 5-Phenyl-1,3,4-thiadiazol | 2 | 2 |
| 21 | 5-Chlor-1,3,4-thiadiazol | 1 | 2 |
| 22 | 5-Chlor-1,3,4-thiadiazol | 2 | 2 |
| 23 | 5-Brom-1,3,4-thiadiazol | 1 | 2 |
| 24 | 5-Brom-1,3,4-thiadiazol | 2 | 2 |
| 25 | 5-Thienyl-1,3,4-thiadiazol | 1 | 2 |
| 26 | 5-Thienyl-1,3,4-thiadiazol | 2 | 2 |
| 27 | 5-Isopentyl-1,3,4-thiadiazol | 1 | 2 |
| 28 | 5-Isopentyl-1,3,4-thiadiazol | 2 | 2 |
| 29 | 5-(2,2-Dimethylpropyl)-1,3,4-thiadiazol | 1 | 2 |
| 30 | 5-(2,2-Dimethylpropyl)-1,3,4-thiadiazol | 2 | 2 |
| 31 | 5-(2-Methylbutyl)-1,3,4-thiadiazol | 1 | 2 |
| 32 | 5-(2-Methylbutyl)-1,3,4-thiadiazol | 2 | 2 |
| 33 | 5-Methylcyclohexyl-1,3,4-thiadiazol | 1 | 2 |
| 34 | 5-Methylcyclohexyl-1,3,4-thiadiazol | 2 | 2 |
| 35 | 5-Methylcyclopropyl-1,3,4-thiadiazol | 1 | 2 |
| 36 | 5-Methylcyclopropyl-1,3,4-thiadiazol | 2 | 2 |

4. Verbindungen nach Anspruch 1, wobei Het, n und m die folgenden Bedeutungen aufweisen:
| Nr | Het | n | m |
|---|---|---|---|
| 1 | 5-Methyl-1,3,4-thiadiazol | 1 | 2 |
| 2 | 5-Methyl-1,3,4-thiadiazol | 2 | 2 |
| 3 | 5-Ethyl-1,3,4-thiadiazol | 1 | 2 |
| 4 | 5-Ethyl-1,3,4-thiadiazol | 2 | 2 |
| 5 | 5-Isopropyl-1,3,4-thiadiazol | 1 | 2 |
| 6 | 5-Isopropyl-1,3,4-thiadiazol | 2 | 2 |
| 9 | 5-Isobutyl-1,3,4-thiadiazol | 1 | 2 |
| 10 | 5-Isobutyl-1,3,4-thiadiazol | 2 | 2 |

5. Verbindungen nach einem der Ansprüche 1 bis 5, wobei n in racemischer Form, isomerenrein oder in Gemischen daraus gleich 1 ist.

6. Verwendung von heterozyklischen Trifluoralkenyl-Verbindungen der Formel (I)
Het-S(O)ₙ-(CH₂)ₘ-CF=CF₂ (I)
wobei:
- Het für eine aromatische heterozyklische Gruppe, gegebenenfalls substituiert, steht, die aus Folgenden ausgewählt ist: 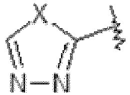
- X für ein Schwefelatom steht;
- n für 1 oder 2 steht;
- m für 1 oder 2 steht,
zur Kontrolle von Nematoden.

7. Verwendung von heterozyklischen Trifluoralkenyl-Verbindungen der Formel (I) nach einem der Ansprüche 2 bis 6 zur Kontrolle von Nematoden, vorzugsweise zur Kontrolle von Pratylenchus spp, Globodera spp, Heterodera spp, Meloidogyne spp, Aphelenchoides spp, Radopholus Similis, Ditylenchus Dipsaci, Tylenchulus Semipenetrans, Longidorus spp, Xiphinema spp, Trichodorus spp, Bursaphelenchus spp, auf kurative und/oder präventive Art und Weise.

8. Agronomische Zusammensetzungen, umfassend eine oder mehrere Verbindungen der Formel (I) nach einem oder mehreren der Ansprüche 1 bis 6 in Kombination mit einem Lösungsmittel und/oder einem Feststoff, einem flüssigen oder verflüssigtem Verdünnungsmittel, gegebenenfalls einem oder mehreren Tensiden und anderen agronomisch annehmbaren Co-Formulierungsmitteln.

9. Agronomische Zusammensetzungen nach Anspruch 8, wobei die Konzentration der aktiven Verbindung der Formel (I) im Bereich von 0,1 bis 90 Gew.-%, bezogen auf das Gesamtgewicht der Zusammensetzung, vorzugsweise im Bereich von 0,5 bis 90 Gew.-% liegt.

10. Agronomische Zusammensetzungen nach einem oder mehreren der Ansprüche 8 bis 9, umfassend mindestens eine Verbindung der Formel (I) und mindestens einen zweiten Wirkstoff, ausgewählt aus Insektiziden, Akariziden, Nematiziden, die nicht jene der Formel (I) sind, Herbiziden, Fungiziden, Bakteriziden, Düngemitteln und Bio-Stimulatoren.

11. Agronomische Zusammensetzungen nach Anspruch 10, wobei das Gewichtsverhältnis zwischen der Verbindung der Formel (I) und der weiteren Wirkstoffe im Bereich von 1:100 bis 100:1, vorzugsweise von 1:10 bis 10:1 liegt.

12. Agronomische Zusammensetzungen nach Anspruch 10, wobei die Konzentration des Wirkstoffs im Bereich von 0,5 bis 90 Gew.-%, bezogen auf das Gesamtgewicht der Zusammensetzung, vorzugsweise im Bereich von 5 bis 90 Gew.-% liegt.

13. Verwendung der agronomischen Zusammensetzungen nach einem oder mehreren der Ansprüche 8 bis 12 zur Kontrolle von Nematoden.

14. Verwendung der agronomischen Zusammensetzungen nach Anspruch 13 durch Anwendung der Zusammensetzung auf die Nutzpflanze über deren Blätter oder auf den Boden mittels Fertigation oder Aufnahme in den Boden oder durch Samenpflege.

15. Verfahren zur Kontrolle von Nematoden in kultivierten Bereichen, das aus dem Anwenden wirksamer und nicht phytotoxischer Dosen von agronomischen Zusammensetzungen nach einem der vorstehenden Ansprüche 8 bis 12, umfassend mindestens eine Verbindung der Formel (I) und gegebenenfalls einen oder mehrere weitere bekannte, damit kompatible Wirkstoffe, auf einen beliebigen Teil der zu schützenden Pflanzen besteht.

## Revendications

1. Composés de trifluoroalcényle hétérocycliques ayant la formule (I)
Het-S(O)ₙ-(CH₂)ₘ-CF=CF₂ (I)
dans lesquels :
- Het représente un groupe hétérocyclique aromatique, substitué, sélectionné parmi : 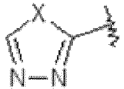
- X représente un atome de soufre ;
- n représente 1 ou 2 ;
- m représente 1 ou 2,
dans lequel les substituants de l'hétérocycle Het sont sélectionnés parmi l'halogène ; un alkyle en C₁-C₆ ; un haloalkyle en C₁-C₆ ; un cycloalkyle en C₃-C₆ ; un formyle ; un aryle facultativement substitué ; un benzyle ; un groupe hétérocyclique penta- ou hexa-atomique aromatique, facultativement benzocondensé ou hétérobicyclique, contenant au moins un hétéroatome sélectionné parmi l'oxygène, le soufre, l'azote ou un N-oxyde facultativement substitué, de préférence sélectionné parmi l'oxygène, le soufre ou l'azote.

2. Les composés selon la revendication 1, dans lesquels
- Het, substitué, représente : 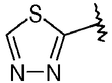
- le substituant du groupe hétérocyclique est un groupe alkyle en C₁-C₆;
- m est égal à 2.

3. Composés selon la revendication 1, dans lesquels Het, n et m ont les significations suivantes :
| N° | Het | n | m |
|---|---|---|---|
| 1 | 5-méthyl-1,3,4-thiadiazole | 1 | 2 |
| 2 | 5-méthyl-1,3,4-thiadiazole | 2 | 2 |
| 3 | 5-éthyl-1,3,4-thiadiazole | 1 | 2 |
| 4 | 5-éthyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 7 | 5-propyl-1,3,4-thiadiazole | 1 | 2 |
| 8 | 5-propyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |
| 11 | 5-cyclopropyl-1,3,4-thiadiazole | 1 | 2 |
| 12 | 5-cyclopropyl-1,3,4-thiadiazole | 2 | 2 |
| 13 | 5-cyclohexyl-1,3,4-thiadiazole | 1 | 2 |
| 14 | 5-cyclohexyl-1,3,4-thiadiazole | 2 | 2 |
| 15 | 5-trifluorométhyl-1,3,4-thiadiazole | 1 | 2 |
| 16 | 5-trifluorométhyl-1,3,4-thiadiazole | 2 | 2 |
| 17 | 5-difluorométhyl-1,3,4-thiadiazole | 1 | 2 |
| 18 | 5-difluorométhyl-1,3,4-thiadiazole | 2 | 2 |
| 19 | 5-phényl-1,3,4-thiadiazole | 1 | 2 |
| 20 | 5-phényl-1,3,4-thiadiazole | 2 | 2 |
| 21 | 5-chloro-1,3,4-thiadiazole | 1 | 2 |
| 22 | 5-chloro-1,3,4-thiadiazole | 2 | 2 |
| 23 | 5-bromo-1,3,4-thiadiazole | 1 | 2 |
| 24 | 5-bromo-1,3,4-thiadiazole | 2 | 2 |
| 25 | 5-thiényl-1,3,4-thiadiazole | 1 | 2 |
| 26 | 5-thiényl-1,3,4-thiadiazole | 2 | 2 |
| 27 | 5-isopentyl-1,3,4-thiadiazole | 1 | 2 |
| 28 | 5-isopentyl-1,3,4-thiadiazole | 2 | 2 |
| 29 | 5-(2,2-diméthylpropyl)-1,3,4-thiadiazole | 1 | 2 |
| 30 | 5-(2,2-diméthylpropyl)-1,3,4-thiadiazole | 2 | 2 |
| 31 | 5-(2-méthylbutyl)-1,3,4-thiadiazole | 1 | 2 |
| 32 | 5-(2-méthylbutyl)-1,3,4-thiadiazole | 2 | 2 |
| 33 | 5-méthylcyclohexyl-1,3,4-thiadiazole | 1 | 2 |
| 34 | 5-méthylcyclohexyl-1,3,4-thiadiazole | 2 | 2 |
| 35 | 5-méthylcyclopropyl-1,3,4-thiadiazole | 1 | 2 |
| 36 | 5-méthylcyclopropyl-1,3,4-thiadiazole | 2 | 2 |

4. Les composés selon la revendication 1, dans lesquels Het, n et m ont les significations suivantes :
| N° | Het | n | m |
|---|---|---|---|
| 1 | 5-méthyl-1,3,4-thiadiazole | 1 | 2 |
| 2 | 5-méthyl-1,3,4-thiadiazole | 2 | 2 |
| 3 | 5-éthyl-1,3,4-thiadiazole | 1 | 2 |
| 4 | 5-éthyl-1,3,4-thiadiazole | 2 | 2 |
| 5 | 5-isopropyl-1,3,4-thiadiazole | 1 | 2 |
| 6 | 5-isopropyl-1,3,4-thiadiazole | 2 | 2 |
| 9 | 5-isobutyl-1,3,4-thiadiazole | 1 | 2 |
| 10 | 5-isobutyl-1,3,4-thiadiazole | 2 | 2 |

5. Les composés selon n'importe lesquelles des revendications 1 à 5, dans lesquels n est égal à 1, sous forme racémique, isomériquement pur, ou leurs mélanges.

6. Utilisation de composés de trifluoroalcényle hétérocycliques ayant la formule (I)
Het-S(O)ₙ-(CH₂)ₘ-CF=CF₂ (I)
dans lesquels :
- Het représente un groupe hétérocyclique aromatique, facultativement substitué, sélectionné parmi : 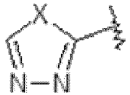
- X représente un atome de soufre ;
- n représente 1 ou 2 ;
- m représente 1 ou 2
pour la lutte contre les nématodes.

7. Utilisation de composés de trifluoroalcényle hétérocycliques ayant la formule (I) selon n'importe lesquelles des revendications 2 à 6 pour la lutte contre les nématodes, de préférence pour la lutte contre Pratylenchus spp, Globodera spp, Heterodera spp, Meloidogyne spp, Aphelenchoides spp, Radopholus Similis, Ditylenchus Dipsaci, Tylenchulus Semipenetrans, Longidorus Spp, Xiphinema Spp, Trichodorus spp, Bursaphelenchus spp, d'une nature curative et/ou préventive.

8. Compositions agronomiques comprenant un ou plusieurs composés ayant la formule (I) selon une ou plusieurs des revendications 1 à 6, en combinaison avec un solvant et/ou un diluant solide, liquide ou liquéfié, facultativement un ou plusieurs tensioactifs et d'autres co-formulants agronomiquement acceptables.

9. Les compositions agronomiques selon la revendication 8, dans lesquelles la concentration en composé actif ayant la formule (I) va de 0,1 à 90 % en poids par rapport au poids total de la composition, de préférence de 0,5 à 90 % en poids.

10. Les compositions agronomiques selon une ou plusieurs des revendications 8 à 9, comprenant au moins un composé ayant la formule (I) et au moins une deuxième matière active sélectionnée parmi des insecticides, des acaricides, des nématicides autres que ceux ayant la formule (I), des herbicides, des fongicides, des bactéricides, des engrais et des bio-stimulants.

11. Les compositions agronomiques selon la revendication 10, dans lesquelles le rapport pondéral entre le composé ayant la formule (I) et les autres matières actives va de 1/100 à 100/1, de préférence de 1/10 à 10/1.

12. Les compositions agronomiques selon la revendication 10, dans lesquelles la concentration en matière active va de 0,5 à 90 % en poids par rapport au poids total de la composition, de préférence de 0,5 à 90 % en poids.

13. Utilisation des compositions agronomiques selon une ou plusieurs des revendications 8 à 12, pour la lutte contre les nématodes.

14. Utilisation des compositions agronomiques selon la revendication 13 par application de la composition sur la culture par l'intermédiaire des feuilles, ou au sol par fertigation, ou incorporation dans le sol, ou par le soin des graines.

15. Un procédé de lutte contre les nématodes dans des zones cultivées, qui consiste à appliquer, sur une partie quelconque des plantes à protéger, des dosages efficaces et non phytotoxiques de compositions agronomiques selon n'importe lesquelles des revendications précédentes 8 à 12, comprenant au moins un composé ayant la formule (I), et, facultativement, un ou plusieurs autres matières actives connues compatibles avec ceux-ci.
